# EUROPEAN PATENT SPECIFICATION

(11) **EP 2 834 215 B1**
(45) Date of publication and mention of the grant of the patent: **26.04.2017**
(21) Application number: 13714265.9
(22) Date of filing: 02.04.2013
(51) Int. Cl.: C07C 211/51, C07C 235/12, C07D 235/26

(54) **PROCESS FOR MAKING BENDAMUSTINE**
VERFAHREN ZUR HERSTELLUNG VON BENDAMUSTIN
PROCÉDÉ DE FABRICATION DE BENDAMUSTINE

(30) Priority: 03.04.2012 WO PCT/EP2012/056096
(43) Date of publication of application: 11.02.2015
(73) Proprietor: Synthon BV, 6545 CM Nijmegen (NL)
(72) Inventor: BARTOS, Petr, 67817 Blansko (CZ); BENOVSKY, Petr, 67817 Blansko (CZ); BARTL, Jiri, 67817 Blansko (CZ)
(74) Representative: Tejedor Vinent, Henar
(86) International application number: PCT/EP2013/056920
(87) International publication number: WO 2013/150020

(56) References cited:
- US-A1- 2011 190 509
- JIAN CHEN ET AL: "Discovery of a Novel, Efficient, and Scalable Route to Bendamustine Hydrochloride: The API in Treanda", ORGANIC PROCESS RESEARCH & DEVELOPMENT, 13 August 2011 (2011-08-13), XP055021034, ISSN: 1083-6160, DOI: 10.1021/op200176f
- GAO LI-MEI ET AL: "Synthesis of 5-[bis(2-chloroethyl)amino]-1-methyl-1H-be nzimidazole-2- butanoic acid hydrochloride (bendamustine hydrochloride)", ZHONGGUO XIN YAO ZAZHI - CHINESE NEW DRUGS JOURNAL, GAI-KAN BIANJIBU, BEIJING, CN, vol. 16, no. 23, 2007, pages 1960-1961, XP008115772, ISSN: 1003-3734 cited in the application

## Description

The invention deals with a novel process and intermediates for making the pharmaceutically useful product bendamustine or a pharmaceutically acceptable salt thereof.

### OVERVIEW OF THE PRIOR ART

### Bendamustine, 4-[5-[bis(2-chloroethyl)amino]-1-methyl-2-benzimidazolyl]butyric acid of formula (1)

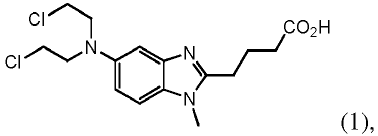

, is a cytostatic agent currently approved, in a form of a hydrochloride salt, for treatment of various cancer diseases, e.g. chronic lymphocytic leukemia. It is marketed in the form of a lyophilized powder for intravenous injection, e.g., under the brand name Ribomustin.

Bendamustine, including bendamustine hydrochloride, was first disclosed in DD 34727. Bendamustine hydrochloride may exist, in solid state, in various polymorphic forms, which are disclosed, e.g. in WO 2009/120386. The hydrochloride product disclosed in DD 34727 is a monohydrate.

The original process for making bendamustine in DD 34727 comprises the following synthetic pathway:

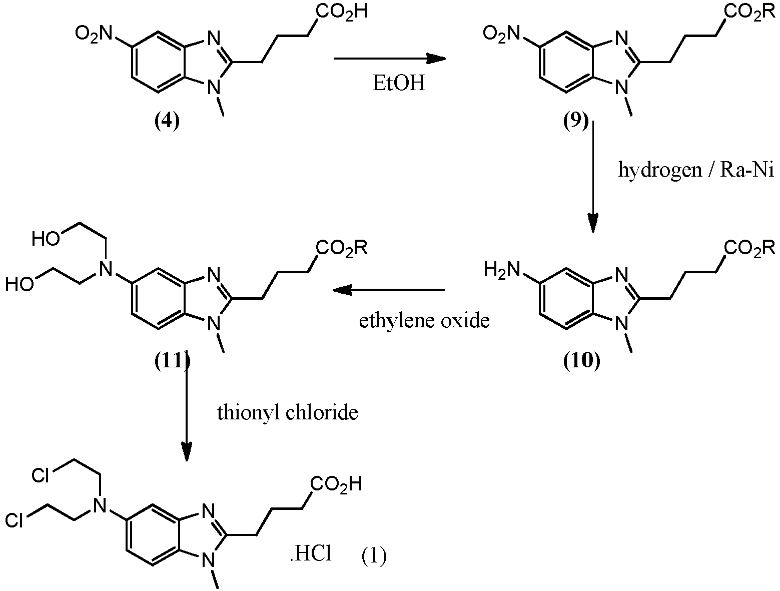

The group R in the above process is an ethyl group.

The last step of the above process was subsequently technologically improved in DD 159877.

Without providing any experimental detail, DD 34727 also teaches that the starting compound of formula (4) for the above process may be prepared from 2-methylamino-5-nitroaniline of formula (2) and glutaric acid anhydride. The obtained anilide of formula (3) is cyclized in diluted hydrochloric acid.

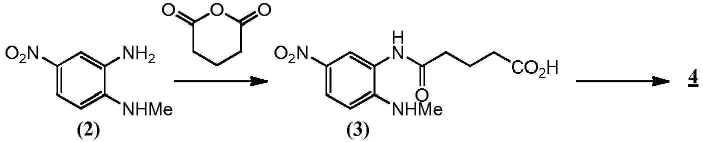

Li-Mei et al., in Zhongguo Xinyao Zazhi, Chinese Journal of New Drugs (2007), 16(23), 1960-1961, disclose a process for the preparation of bendamustine hydrochloride in a total yield of 33.5%, which also involves reacting the compound of formula (10) with ethylene oxide to give compound (11). Starting from 2,4-dinitro-1-chlorobenzene, compound (11) is obtained in an overall yield of about 40%.

IP.com Journal 2009, 9(7B), 21 discloses a process for the preparation of ethyl-4-[5-[bis(2-hydroxyethyl)amino]-1-methylbenzimidazol-2-yl]butanoate (11) [R=Et], wherein the corresponding compound of formula (10) reacts, instead of ethylene oxide, with 2-halo ethanol in the presence of an inorganic base.

A similar process has been disclosed in WO 2011/079193, wherein the base employed in the reaction of the compound of formula (10) with the 2-haloethanol is an organic base, which is advantageous over inorganic base. The preferred ester group R in the compounds (10) and (11) is the 2-propyl group.

WO 2010/042568 discloses a second basic process for making bendamustine, which is based on providing the compound of formula (5) 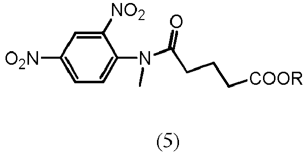 , wherein R is typically a methyl group, by a two step synthesis starting from 2,4-dinitroaniline of formula (6) via the dinitroanilide of formula (7)

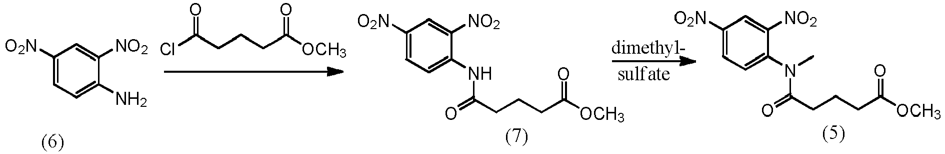

This compound of formula (5) is subjected, at reductive conditions (preferably hydrogenation over a platinum catalyst), to a cyclization reaction forming a compound of formula (8) 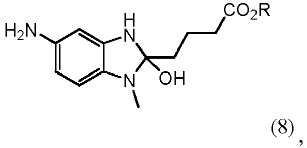 , which subsequently may be dehydrated by a strong acid to yield the compound of formula (10) above. The substituent R in both formulas is a methyl group.

The compound of formula (10) is advantageously subjected to a reductive alkylation with a chloroacetic acid or chloroacetylaldehyde. The reductive agent in the alkylation is suitably a borane or a borohydride. This way, the bendamustine ester of formula (1a) 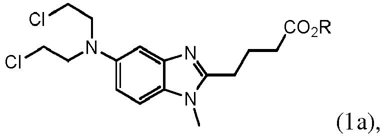 , wherein R is a methyl group, is made directly, without need of forming an intermediate bis-hydroxyethyl compound (11). In the last step of the overall process, the ester (1a) is hydrolyzed by a strong acid.

In any process of making bendamustine, various impurities are formed due to various reactive groups in the molecule.

In particular, a mono-hydroxyethyl impurity of general formula (A) may be formed during the hydroxyethylation reaction on the amine group of the compound (10).

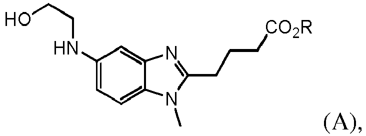

The formation of impurities reduces the overall yield of the product and also makes the process more expensive due to a need of using elaborated purification procedures.

Despite its longstanding use, it is apparent that there exist only few attempts to improve the process of making bendamustine. Thus, there is an objective need to find an alternative, notably industrial scale process of making bendamustine, which process would advantageously be less expensive and safer than the ones presently known from the prior art.

### BRIEF DESCRIPTION OF THE PRESENT INVENTION

The subject of the present invention is a novel synthetic route to intermediates involved in the synthesis of bendamustine of formula (1) as well as of salts and esters thereof. The approach is based on a novel use of a compound of formula (13) below as the starting material in a synthetic transformation leading to bendamustine, or a pharmaceutically acceptable salt thereof.

In a first aspect, the invention provides a process for making a compound of formula (11), or a salt thereof, 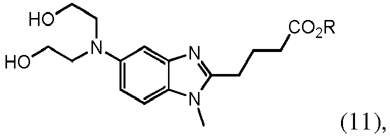 wherein R is hydrogen or a C1-C4 alkyl group,
said process comprising the following steps:
a] providing the compound of formula (13), preferably by reaction of the compound of formula (12) with methylamine, 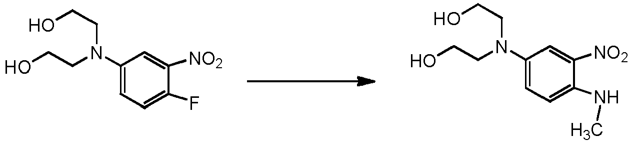 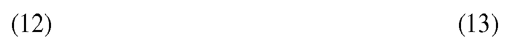
b] reduction of the compound of formula (13), preferably by hydrogen under catalysis by a transition metal, to an amino compound of formula (14), 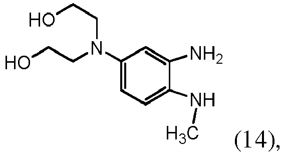
c] condensation of the compound of formula (14) with glutaric acid anhydride, or a functional analogue thereof, providing a tertiary alcohol compound of formula (15) 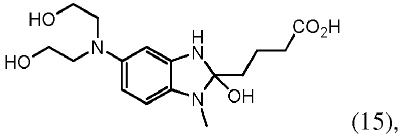 and/or any tautomeric forms thereof according to formula (14A) or (14B) 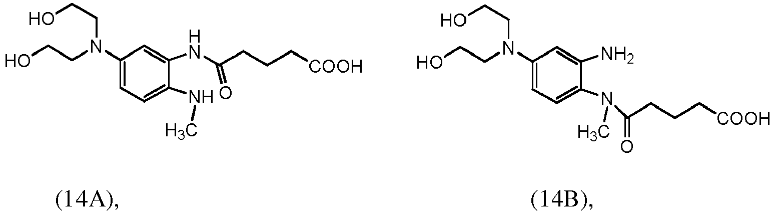
d] dehydratation and, optionally, esterification of the product of the step c), preferably in the presence of a strong acid, to yield the compound of formula (11).

In a particular aspect, the above process sequence leading to a compound of formula (11) further comprises a subsequent step of converting the compound of formula (11) to bendamustine, a salt thereof or an ester thereof, conventionally by reaction with thionyl chloride, followed by ester hydrolysis and salt formation using hydrochloric acid.

In yet another aspect, the process sequence of the above steps a) to c) or, optionally, of the above steps a) to d), is performed without isolation or purification of intermediates.

The compounds of formula (14), (14A), (14B) and (15), the above processes of making them, and the use thereof as a starting material for making compounds of formula (11) and/or bendamustine of formula (1), or a pharmaceutically acceptable salt thereof, form next particular aspects of the present invention.

In another aspect, the present invention also relates to the use of the compound of formula (12) and (13) for making bendamustine of formula (1), or a pharmaceutically acceptable salt thereof.

### DETAILED DESCRIPTION OF THE PRESENT INVENTION

The present invention provides an alternative route for making bendamustine of formula (1) based on employing an otherwise known, but hitherto unused starting material for making bendamustine, namely the compound of formula (13). In an important advantageous aspect, this starting material already comprises the bis-2-hydroxyethylamino moiety, which is a key element of the bendamustine structure. This avoids the need of building in said moiety in later steps of bendamustine synthesis, which need to be carried out under regulations prescribed for pharmaceutical production. Using toxic and explosive oxirane in pharmaceutical production is accompanied with a need of investing into expensive safety measures. Instead, the bis-hydroxyethylamino- material is obtained as a raw chemical in a specialized chemical plant. Furthermore, it can be obtained in a sufficient degree of purity, or it may be suitably purified, well before its use in constructing the benzimidazole skeleton. As a result, the process of the invention avoids forming impurities of formula (A) or structurally related compounds, which may be otherwise present in the final bendamustine product due to incomplete hydroxyethylation reaction in later steps, as mentioned above.

Moreover, as will be shown below, the novel synthesis of the key intermediate (11) can be industrially performed without isolation of any intermediates to a large extent, which may contribute significantly to lower the burden on process resources, starting from (12) in an overall isolated yield of more than 85%, and in some embodiments more than 90%.

The process of the present invention is primarily focused on making a compound of formula (11), wherein R is hydrogen or a C1-C4 alkyl group, which is the last intermediate in making bendamustine of formula (1) according to known literature procedures. The process according to various aspects of the present invention is shown in the scheme below.

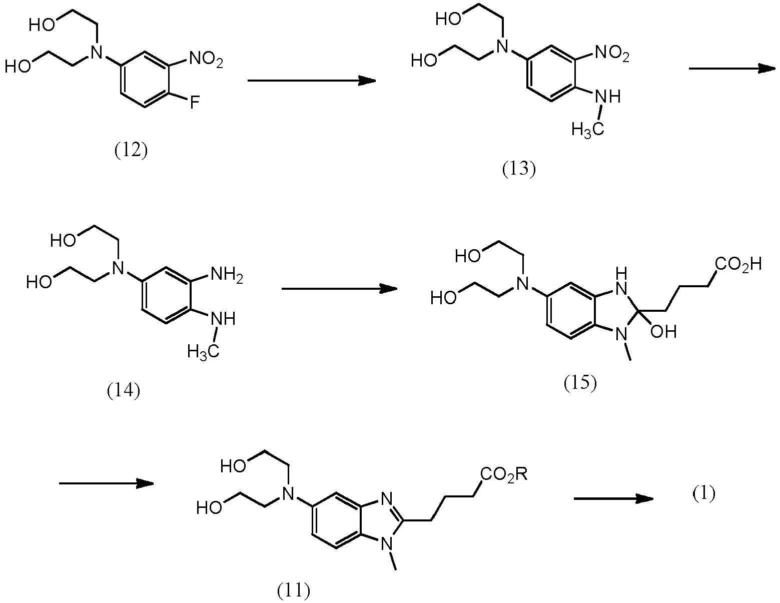

Thus, the first step of the process of the present invention includes providing the compound of formula (13). This compound is known and has been used as a chemical in hair-dye industry; accordingly, it may be commercially available. Alternatively, it may be produced by a suitable process (see, e.g., US 3,944,612 or US 5,414,128). In particular, it may be obtained by reaction of 2-nitro-4-bis(2-hydroxyethyl)aminofluorobenzene (12) with methylamine, advantageously with aqueous methylamine. The aqueous methylamine may advantageously serve as the reaction medium. The corrosive fluoride ion arising from the synthesis may be advantageously trapped by including boric acid in the reaction medium. If desired or advantageous, compound (13) may be isolated from the reaction mixture as such or in the form of a salt, and optionally purified.

In the next step of the process of the invention, compound (13) is reduced to yield the aniline compound of formula (14). The reducing agent is preferably hydrogen under catalysis of a transition metal, e.g. Pd, Pt, Ni, or Co. The hydrogen reacts at ambient or at an enhanced pressure. A suitable solvent or reaction medium is water, a C 1-C4 aliphatic alcohol or a mixture of both. Other suitable reducing agents may be, without limitation, a borane, a hydride, a dithionite, zinc or magnesium in the presence of a strong acid, etc. A preferred solvent is a C1-C4 aliphatic alcohol. If desired or advantageous, compound (14) may be isolated from the reaction mixture as such or in the form of a salt, and optionally may be purified. It is however preferred that the compound of formula (14) is not isolated from the reaction mixture.

In the following step of the process of the invention, compound (14) is subjected to a condensation reaction with glutaric acid anhydride or a functional analogue thereof, which functional analogue represents another suitable donor of the desired butyric acid moiety (e.g. an ester chloride of glutaric acid). The condensation reaction typically proceeds in an inert solvent, with may advantageously be a C1-C4 aliphatic alcohol, e.g. methanol or ethanol. The condensation reaction primarily acylates both the primary and the secondary amino groups of compound (13) under formation of compounds (14A) and/or (14B) 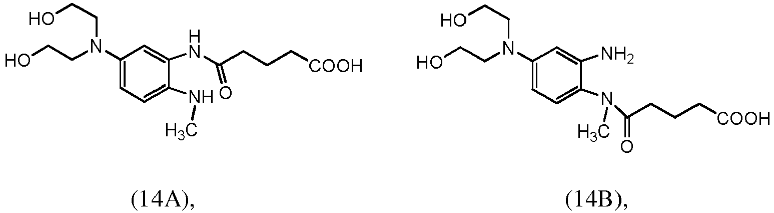 , which can subsequently cyclize under formation of an imidazolyl ring of the compound (15). Such cyclization is sometimes reversible in solutions, so that the reaction product may be an equilibrium mixture of the compounds (15), (14A) and (14B). However, any of the compounds is equally able to undergo the dehydration reaction disclosed below, providing thereby the desired compound (11).

The formation of bis-acylated compound (14C) 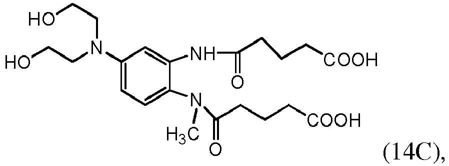 is undesired and may be suppressed by using a near-stoichiometric amount of the glutaric acid anhydride (advantageously 1.0-1.5 molar equivalent, more preferably 1.10-1.25 molar equivalent, with respect to compound (14)) and its addition, either as such or in a solution in a suitable inert solvent, in portions over time.

An excess of glutaric acid anhydride may also cause formation of esters of glutaric acid according to formulae (15A) and/or (15B) 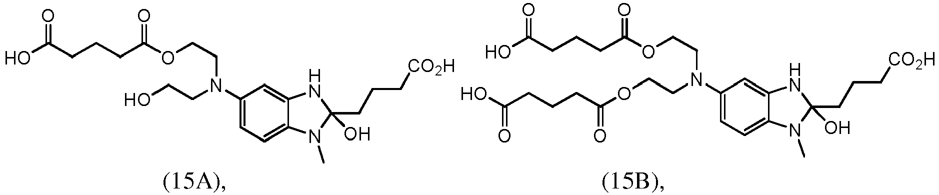 as side products ; their presence in the compound (15) is however allowable as such esters may be easily saponified in later steps under liberation the hydroxyethyl group again.

Alternatively, the OH-groups in the compound (14) may be protected against such side reactions by suitable OH-protective groups to assure more economic use of the glutaric acid aldehyde, although this is not preferred. Details of said alternative are described below.

If desired or advantageous, compound (15) may be isolated from the reaction mixture as such or in the form of a salt and/or an ester, and optionally purified. Suitable acid addition salts include salts with hydrochloric, hydrobromic, sulfuric, nitric or phosphoric acid as well as with a suitable organic acid. Suitable esters include esters with C1-C4 aliphatic alcohols and/or with C1-C10 carboxylic acids. It is however preferred that the compound (15) is not isolated from the reaction mixture and the solution thereof (incl. its tautomers (14A) and/or (14B)) is used in the next step.

In a last step, the compound of formula (15) (or, as an alternative, any of the compounds (14A) or (14B)) is subjected to a dehydration reaction forming the compound of formula (11). A useful dehydration agent is, e.g., a strong acid, preferably a strong inorganic acid, most preferably hydrochloric acid or sulfuric acid. Other dehydration agents, for instance a strongly acidic ion-exchange resin, may be used as well. If a C1-C4 alcohol is used as a reaction medium, an ester of formula (11) [R = C1-C4 alkyl] is formed. Such formation is advantageous because of an easy isolation of such ester from the reaction medium, while the acid (11) [R=H] sometimes is isolated with difficulties from the reaction mixture, particularly when a polar medium (such as water) is used. Furthermore, compound (11), wherein R is not hydrogen, can advantageously be used in further steps in making bendamustine as such R group protects the carboxy group against undesired side reactions and can be removed in the final reaction step. The acid (11) [R=H] may be made, whenever necessary, by saponification of the ester (11) [R = C1-C4 alkyl] with a metal hydroxide, typically sodium hydroxide, in water.

Thus, as a result, the compound of formula (11), wherein R is hydrogen or a C1-C4 alkyl group, is obtained easily from commercially available starting compound (12) or (13) without a need of using toxic and explosive oxirane. What is also important is that the whole sequence may be performed in a single production step, i.e. without isolation or purification of intermediates. In a typical non-limiting arrangement of such process:
a) compound (12) reacts with a methylamine aqueous solution, preferably in the presence of boric acid, at an enhanced temperature (40-90°C, preferably 50-80°C); advantageously the reaction is performed in a closed pressure vessel;
b) the reaction mixture is diluted with an alcohol, typically methanol or ethanol, optionally concentrated by a distillation in order to remove excess of used methylamine and water, transferred into a pressure reactor and hydrogenated with hydrogen in the presence of a suitable hydrogenation catalyst, e.g. a palladium catalyst, typically at an ambient or near ambient temperature (20-40°C);
c) after removal of the catalyst, or in some embodiments even without removal of the catalyst, the glutaric acid anhydride is added to the reaction mixture, either as such or in the form of a solution in an inert solvent, e.g. an ester solvent such as ethyl acetate or methyl acetate, within a suitable time, advantageously within several hours (in some embodiments within 8 hours, preferably within 6 hours, most preferably within 1-3 hours), typically at an ambient or near ambient temperature (20-40°C);
d) the mixture, from with the catalyst has been already removed, is acidified with an acid, preferably hydrochloric acid or sulfuric acid, and heated at an enhanced temperature (40-80°C, preferably 50-70°C) for one or more hours.

In such an arrangement, compound (11), wherein R is methyl or ethyl, is formed, which may be optionally isolated from the reaction mixture. Similarly, compound (11) having an alkyl group R other than methyl or ethyl may be prepared as well.

In an analogous way, the process may be performed with compound (13) as the starting material; the first reaction step is avoided then.

The compound of formula (11), wherein R is hydrogen or advantageously a C1-C4 alkyl group, is then converted to bendamustine of formula (1) or a pharmaceutically acceptable salt thereof by processes known in the art. In one embodiment, the ester compound (11) is subjected to a chlorination reaction by a suitable chlorinating agent, which may be, e.g. chlorine, thionyl chloride, phosphorus trichloride, phosphorus pentachloride, phosphorus oxychloride, fosgene, difosgene, or oxalylchloride, etc. The reaction typically proceeds in an inert solvent, e.g. in a hydrocarbon or a chlorinated hydrocarbon solvent. From 1 to 6 molar equivalents of the chlorinating agent are typically used. The product of said conversion is corresponding bendamustine ester (1a), which may be isolated from the reaction mixture and optionally purified.

After chlorination, the ester group, if present, is cleaved by an aqueous acid. Thereby, a salt of bendamustine with the corresponding acid is formed and may be as such isolated from the reaction mixture. Advantageously, bendamustine hydrochloride may be obtained this way.

In a specific aspect of the invention, the present application provides novel compounds of formulae (15), (14A) and (14B) useful as intermediates for making compounds of formula (11) and, subsequently, for preparing bendamustine of formula (1) or a pharmaceutically acceptable salt thereof. The compounds of formula (15), (14A) and (14B) include also esters with a C1-C4 aliphatic alcohol and/or with a C1-C10 carboxylic acid as such compounds are, in essence, equally suitable for purposes of the present invention. The compounds of formula (15), (14A) and (14B) and the above functional analogues thereof also include acid addition salts with an inorganic or organic acid, e.g., with hydrochloric, hydrobromic, phosphoric, sulfuric, nitric, acetic, formic, oxalic, maleic, fumaric, methanesulfonic, benzenesulfonic, or toluenesulfonic acid, etc.

Accordingly, the present invention also includes the use of any of the compounds of formula (15), (14A) and (14B), incl. mixtures thereof, for making bendamustine of formula (1). Said use is advantageously represented by a process, in which any of the compounds of formula (15), (14A) and (14B), incl. mixtures thereof, is converted into the compound of formula (11), wherein R is H or a C1-C4 alkyl group. Said compound (11) is then converted to bendamustine of formula (1) or a pharmaceutically acceptable salt thereof. Details of the corresponding process steps have been already disclosed above.

In a specific aspect of the invention, the present application also provides a novel compound of formula (14) useful as an intermediate in making compounds of formula (11) and, subsequently, for preparing bendamustine of formula (1) or a pharmaceutically acceptable salt thereof. The compound of formula (14) includes also acid addition salts with an inorganic or organic acids, e.g., with hydrochloric, hydrobromic, phosphoric, sulfuric, nitric, acetic, formic, oxalic, maleic, fumaric, methanesulfonic, benzenesulfonic, or toluenesulfonic acid, etc. In a yet specific aspect, the compound (14) also includes esters of (14) with glutaric acid.

As already discussed above, the compound of formula (14) may be advantageously produced by a process sequence comprising
a] providing the compound of formula (13), preferably by reaction of a compound of formula (12) with methylamine under optional presence of boric acid; and
b] reduction of the compound of formula (13), preferably by hydrogen under catalysis by a transition metal, to the compound of formula (14).

Details of said process sequence have been provided above.

Accordingly, the present invention also includes the use of the compound of formula (14) for making bendamustine of formula (1). Said use is advantageously represented by a process, in which the compound of formula (14) is converted into a compound of formula (11), wherein R is H or a C1-C4 alkyl group. Said compound of formula (11) is then converted to bendamustine of formula (1) or a pharmaceutically acceptable salt thereof. Details of the corresponding process steps have been already disclosed above.

In an alternative to the process disclosed above, a suitable starting material may also be a compound of general formula (12.1), wherein X is a OH-protective group. Such OH-protective group protects both OH-groups in the bis-hydroxyethylamino side chain of compound (12) and its subsequent synthetic intermediates (13.1), (14.1) and (15.1) against side reactions, and is removed after obtaining the compound of formula (11.1), e.g. before or during the chlorination reaction.

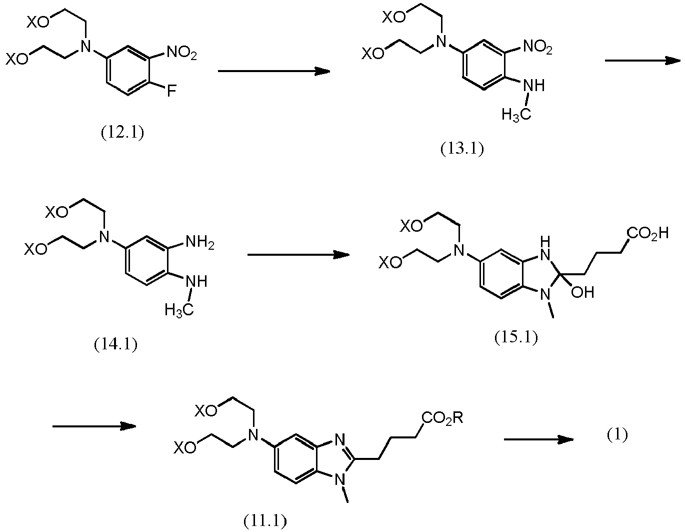

Any suitable OH-protective group X may be used. Advantageously it may be selected from those, which are listed in the publication of Wuts, Peter G.M., Greene, Theodora W.: Greene's Protective Groups in Organic Synthesis, 4th Ed., John Wiley et Sons, 2006. Non-limiting examples are methyl group, methoxymethyl group, benzyl group, tetrahydropyranyl group, a silyl group, and trityl group, etc.

The OH-protective group may be introduced and removed by any suitable process known in the art. The present inventors surprisingly have found that despite the anticipated reactivity of the hydroxyl groups, no OH-protective groups are required in order to carry out the transformation of compound (12) into compound (11) without isolation of any intermediates and in a high overall yield under process conditions as disclosed in this application.

Compound (11) can optionally be re-crystallized from a suitable organic solvent, e.g. ethyl acetate or acetonitrile.

Bendamustine, typically bendamustine hydrochloride, produced according to the processes of the present invention may be advantageously provided in an isolated, typically solid and crystalline form, details of which procedures are known in the art. In particular, it may be obtained in a form of bendamustine hydrochloride monohydrate.

Accordingly, the obtained bendamustine may be used in pharmaceutical compositions, e.g. for the treatment of various kinds of cancer diseases.

The following examples are intended to illustrate the scope of the present invention but not to limit it thereto.

### EXAMPLES

### Example 1

A 5 ml round-bottomed vial equipped with a magnetic stirring bar and a stopper was charged with the compound of formula (12) (244 mg) and 0.437 ml of 39.5 weight % aqueous solution of methylamine. The mixture was stirred at 70°C for 4.5 hours and cooled to room temperature.

The mixture was diluted with 5 ml of ethanol and transferred into a 25 ml pressure reactor equipped with a magnetic stirring bar. The reactor was charged with 136 mg of Pd/C catalyst (3.9 %), flushed with nitrogen, and pressurized with hydrogen to 60 psi. The mixture was stirred at 23°C at 50-60 psi of hydrogen for 2 hours.

The reactor was flushed with nitrogen and charged, under stirring, with 357 mg of glutaric acid anhydride in portions within 5 hours. The catalyst was filtered off.

The filtrate was acidified with 35% HCl to a pH of about 2 and heated to 60°C for 2.5 hours to produce a solution comprising 90% of the compound of formula (11) (R = ethyl).

### Example 2

A 300 ml, pressure reactor equipped with a magnetic stirring bar was charged with the compound of formula [12] (19.54 g, 0.08 mol), boric acid (1.48 g, 24 mmol), and methylamine aqueous solution (35 ml, 0.4 mol). The mixture was stirred at 800 rpm at 70 °C in the sealed reactor for 6.0 h. The mixture was concentrated on a rotary evaporator at 75 °C at 700 mbar and co-evaporated with methanol (150) ml three times. The evaporation residue was diluted with 150 ml of methanol and transferred into a pressure reactor containing palladium on activated carbon (4.37 g, 1.6 mmol, 3.9 % wet basis) moistened with water (1 ml). The reactor was flushed with nitrogen three times, with hydrogen four times, and pressurized to 50 psi with hydrogen. The reaction mixture was stirred at 900 rpm at 23 °C for 2.5 h under hydrogen atmosphere of 50-55 psi. The reactor was charged with a solution of glutaric anhydride (12.36 g, 0.1 mol, 96 %) in methyl acetate (30 ml) via a syringe over 0.1 h and the mixture was stirred at 1000 rpm at 23 °C for 2.0 h under nitrogen atmosphere. The mixture was filtered over a sintered glass filter and the filtrate was collected in a 500 ml, round bottomed flask. The flask was equipped with a magnetic stirring bar as well as a reflux condenser and charged with hydrochloric acid (16.11 ml, 0.18 mol, 35 %). The reaction mixture was stirred at 700 rpm at 73 °C for 2.5 h and cooled to room temperature over 1.0 h. The *pH* of the mixture was adjusted with concentrated aqueous ammonia solution to the value of 6.0 and the mixture was concentrated at 60 °C at 350-100 mbar on the rotary evaporator. The residue was diluted with water (150 ml) and washed with dichloromethane (200 ml). The *pH* of the aqueous layer was adjusted with concentrated aqueous ammonia solution to the value of 8.5 and the layer was extracted with dichloromethane (200 ml). The extract was concentrated at 60 °C at 950 mbar to one fifth of initial volume and diluted with ethyl acetate (200 ml). The solution was filtered over a sintered glass filter and the filtrate was evaporated at 60 °C at 450-100 mbar to produce [11, R = Me] (24.6 g) in an overall isolated yield of 92%.

### Characterization

¹H NMR (400 MHz, DMSO-*d6*): δ (ppm) = 2.04 (q, *J* = 7.33 Hz, 2H), 2.48 (t, *J* = 7.33 Hz, 2H), 2.82 (t, *J* = 7.38 Hz, 2H), 3.41 (t, *J* = 6.32 Hz, 4H), 3.58 (t, *J* = 6.34 Hz, 4H), 3.61 (s, 3H), 3.64 (s, 3H), 4.57 (s, b, 2H), 6.75 (dd, *J*₁ = 2.35 Hz, *J*₂ = 8.81 Hz, 1H), 6.89 (d, *J* = 2.27 Hz, 1H), 7.22 (d, *J* = 8.80 Hz, 1H).

### Example 3

A solution of [11, R = Me] (4.0 g, 12 mmol) in dichloromethane (40.0ml) was prepared. A 100 ml, three-necked, round-bottomed flask equipped with a magnetic stirring bar and a reflux condenser was charged with thionyl chloride (3.60 g, 2.20 ml, 30 mmol) and dichloromethane (12.0 ml) to produce a clear solution. The latter was stirred at 500 rpm at 23 °C and the solution of [11, R = Me] was added over 15 min via a syringe pump. The resulting mixture was stirred at 500 rpm at 23 °C for 15 min and then at 35 °C for 3.0 h. An aqueous solution of hydrochloric acid (19.4 %) was prepared by mixing of concentrated hydrochloric acid (4.7 g, 4.0 ml) with water (4.0 g, 4.0 ml) and the solution was charged to the reaction mixture. The mixture was further stirred at 500 rpm at 60 °C for 2.0 h under reduced pressure of 100 mbar and the escaping volatiles were condensed and collected outside the reaction vessel. An aqueous solution of hydrochloric acid (4.0 ml, 5 M) was charged in order to dilute the reaction mixture. The mixture was filtered through diatomaceous earth and the filter cake was washed with aqueous solution of hydrochloric acid (2x 1.0 ml, 5 M). The collected filtrate was treated with activated carbon, the used carbon was filtered off, washed with aqueous solution of hydrochloric acid (2x 1.0 ml, 5M), and the filtrate was collected to a 100 ml, round-bottomed flask equipped with a magnetic stirring bar. The filtrate was stirred and diluted with water (40.0 g, 40.0 ml). The slurry was filtered and the filter cake was washed with water (2x 1.0 ml). The filter cake (3.8 g) was charged to a 25 ml, round-bottomed flask equipped with a magnetic stirring bar containing an aqueous solution of hydrochloric acid (8.5 ml, 5M). The mixture was stirred at 60 °C until the solids were dissolved and activated carbon (0.38 g) was charged. The mixture was stirred at 40 °C for additional 5 min and the suspension was filtered through a diatomaceous earth pad. The filter cake was washed with aqueous solution of hydrochloric acid (2x 1.0 ml, 5M) and the filtrate was collected to a 100 ml, round-bottomed equipped with a magnetic stirring bar. The filtrate was stirred at 23 °C and water (42.0 g, 42.0 ml) was added and the mixture was stirred at 23 °C for additional 1 h. The slurry of crystals was filtered and the filter cake was washed with aqueous solution of hydrochloric acid (2× 3.0 ml, 5M). The filtrate was discarded and the filter cake was dried to produce Bendamustine hydrochloride monohydrate (3.3 g) with an overall isolated yield of 67 % and with a chemical purity of 99.9 % by HPLC peak area normalization.

### Characterization

¹H NMR (400 MHz, DMSO-*d6*): δ (ppm) = 2.05 (q, *J* = 7.51 Hz, 2H), 2.41 (t, *J* = 7.19 Hz, 2H), 3.18 (t, *J* = 7.63 Hz, 2H), 3.79 (m, 8H), 3.90 (s, 3H), 6.95 (d, *J* = 2.31 Hz, 1H), 7.11 (dd, *J*₁ = 2.40 Hz, *J*₂ = 9.20 Hz, 1H), 7.80 (d, *J* = 9.20 Hz, 1H).
Assay H₂O (Karl-Fisher titration): 4.7 %
Assay HCl (argentometric titration): 8.8 %

## Claims

1. A process for making a compound of formula (11) 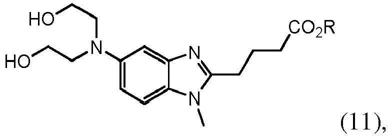 , wherein R is hydrogen or a C1-C4 alkyl group, said process comprising the following steps:
a] providing the compound of formula (13), by reaction of the compound of formula (12), 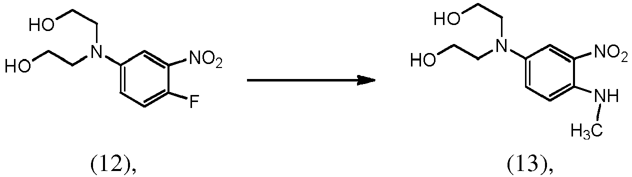
b] reduction of the compound of formula (13) to an amino compound of formula (14), 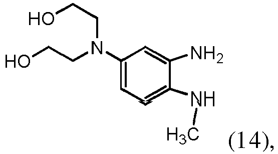
c] condensation of the compound of formula (14) with glutaric acid anhydride or a functional analogue thereof, providing a tertiary alcohol compound of formula (15) and/or any of tautomeric forms thereof of formula (14A) or (14B) ; 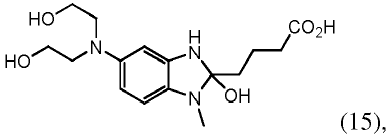 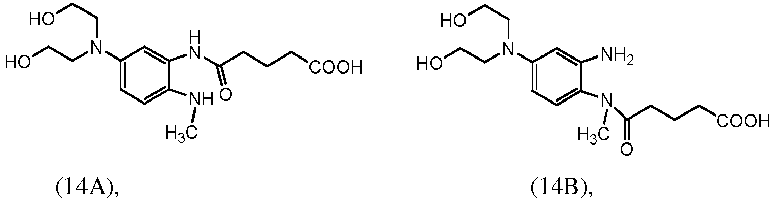 and
d] dehydratation and, optionally, esterification of the product of the step c) to yield the compound of formula (11).

2. The process according to claim 1, further comprising converting the compound of formula (11) to bendamustine of formula (1) 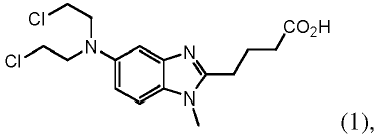 , or a pharmaceutically acceptable salt thereof.

3. The process according to claim 1 or 2, wherein the process sequence of the above steps a) to c) or, optionally, of the above steps a) to d), is performed without isolation or purification of intermediates.

4. The process according to any one of claims 1-3, wherein the reaction in the step a) is performed in the presence of boric acid.

5. The process according to any one of claims 1-4, wherein any of the steps b), c), or d) is performed in a C1-C4 alcoholic reaction medium.

6. The compound of formula (15), (14A), (14B) 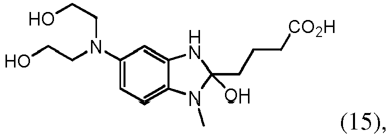 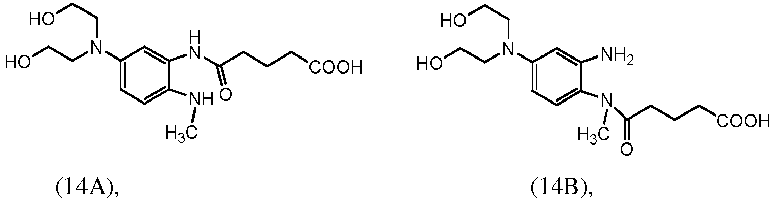 or an ester thereof with a C1-C4 aliphatic alcohol and/or with a C1-C10 carboxylic acid, and/or any acid addition salt thereof.

7. Use of any of the compounds of formula (15), (14A) and (14B) for making bendamustine of formula (1), or a pharmaceutically acceptable salt thereof.

8. The compound of formula (14), 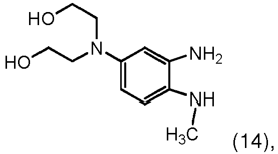 or an acid addition salt thereof.

9. A process of making the compound of formula (14) comprising the steps of
a] providing the compound of formula (13) by reaction of the compound of formula (12) and
b] reduction of the compound of formula (13).

10. Use of the compound of formula (14) for making bendamustine of formula (1), or a pharmaceutically acceptable salt thereof.

11. Use of the compound of formula (12) or (13) for making bendamustine of formula (1), or a pharmaceutically acceptable salt thereof.

12. The process according to any one of claims 1-5, wherein the OH-groups of the bis-hydroxyethylamino side chain of compounds (12), (13), (14), (15), and (11) are protected with a suitable OH-protective group (X), and which OH-protective group is removed at a suitable moment when converting the compound of formula (11) to bendamustine of formula (1), or a pharmaceutically acceptable salt thereof.

13. The use according to claim 10 or 11, wherein the OH-groups of the bis-hydroxyethylamino side chain of compounds (12), (13) or (14) are protected with a suitable OH-protective group (X), and which OH-protective group is removed at a suitable moment.

## Patentansprüche

1. Verfahren zum Herstellen einer Verbindung der Formel (11) 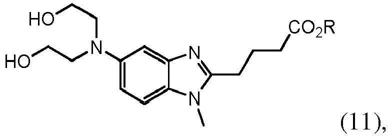 wobei R Wasserstoff oder eine C1-C4-Alkylgruppe ist, wobei das Verfahren die folgenden Schritte umfasst:
a] Bereitstellen der Verbindung der Formel (13) durch Reaktion der Verbindung der Formel (12), 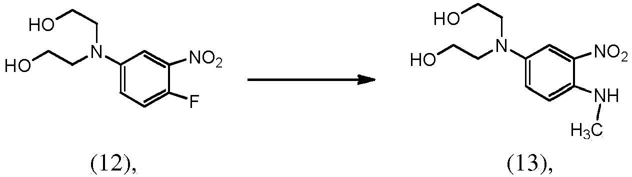
b] Reduktion der Verbindung der Formel (13) zu einer Aminoverbindung der Formel (14), 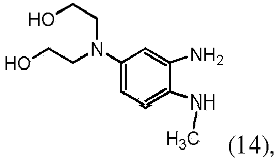
c] Kondensation der Verbindung der Formel (14) mit Glutarsäureanhydrid oder einem funktionellen Analogon davon, was eine tertiäre Alkoholverbindung der Formel (15) und/oder eine beliebige der tautomeren Formen davon der Formel (14A) oder (14B) bereitstellt; 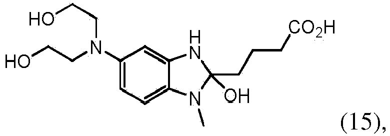 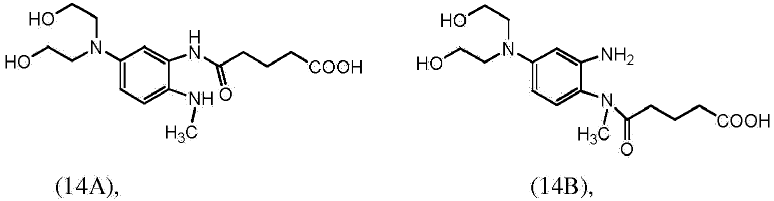 und
d] Dehydratisierung und gegebenenfalls Veresterung des Produkts des Schritts c), um die Verbindung der Formel (11) zu ergeben.

2. Verfahren nach Anspruch 1, weiterhin umfassend Umwandeln der Verbindung der Formel (11) zu Bendamustin der Formel (1) 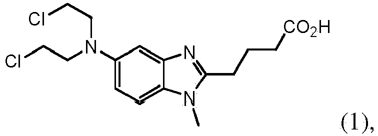 oder einem pharmazeutisch verträglichen Salz davon.

3. Verfahren nach Anspruch 1 oder 2, wobei die Verfahrensabfolge der vorstehenden Schritte a) bis c) oder gegebenenfalls der vorstehenden Schritte a) bis d) ohne Isolierung oder Reinigung von Intermediaten durchgeführt wird.

4. Verfahren nach einem beliebigen der Ansprüche 1-3, wobei die Reaktion im Schritt a) in der Gegenwart von Borsäure durchgeführt wird.

5. Verfahren nach einem beliebigen der Ansprüche 1-4, wobei ein beliebiger der Schritte b), c) oder d) in einem C1-C4-alkoholischen Reaktionsmedium durchgeführt wird.

6. Verbindung der Formel (15), (14A), (14B) 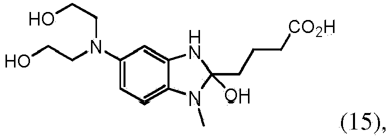 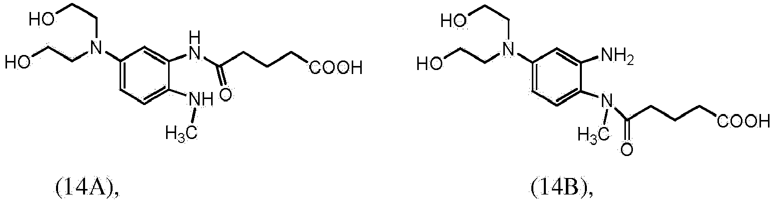 oder ein Ester davon mit einem C1-C4-aliphatischen Alkohol und/oder mit einer C1-C10-Carbonsäure, und/oder ein beliebiges Säureadditionssalz davon.

7. Verwendung einer beliebigen der Verbindungen der Formel (15), (14A) und (14B) zum Herstellen von Bendamustin der Formel (1), oder einem pharmazeutisch verträglichen Salz davon.

8. Verbindung der Formel (14), 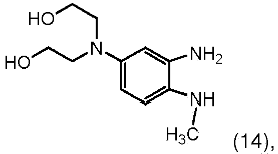 oder ein Säureadditionssalz davon.

9. Verfahren zum Herstellen der Verbindung der Formel (14), umfassend die Schritte
a] Bereitstellen der Verbindung der Formel (13) durch Reaktion der Verbindung der Formel (12) und
b] Reduktion der Verbindung der Formel (13).

10. Verwendung der Verbindung der Formel (14) zum Herstellen von Bendamustin der Formel (1), oder einem pharmazeutisch verträglichen Salz davon.

11. Verwendung der Verbindung der Formel (12) oder (13) zum Herstellen von Bendamustin der Formel (1), oder einem pharmazeutisch verträglichen Salz davon.

12. Verfahren nach einem beliebigen der Ansprüche 1-5, wobei die OH-Gruppen der Bis-hydroxyethylamino-Seitenkette der Verbindungen (12), (13), (14), (15) und (11) mit einer geeigneten OH-Schutzgruppe (X) geschützt sind, und welche OH-Schutzgruppe in einem geeigneten Moment entfernt wird, wenn die Verbindung der Formel (11) zu Bendamustin der Formel (1) oder einem pharmazeutisch verträglichen Salz davon umgewandelt wird.

13. Verwendung nach Anspruch 10 oder 11, wobei die OH-Gruppen der Bishydroxyamino-Seitenkette der Verbindungen (12), (13) oder (14) mit einer geeigneten OH-Schutzgruppe (X) geschützt sind, und welche OH-Schutzgruppe in einem geeigneten Moment entfernt wird.

## Revendications

1. Un procédé de préparation d'un composé de formule (11) 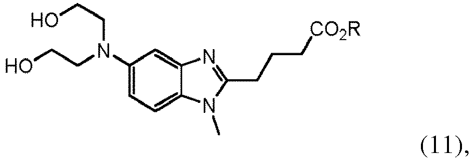 dans laquelle R est un atome d'hydrogène ou un groupe C₁-C₄ alkyle,
lequel procédé comprend les étapes suivantes :
a] obtention du composé de formule (13) par réaction du composé de formule (12), 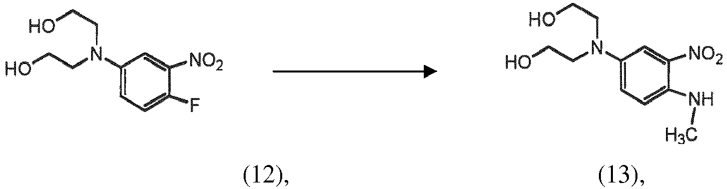
b] réduction du composé de formule (13) en un composé amino de formule (14) 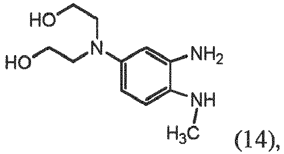
c] condensation du composé de formule (14) avec l'anhydride de l'acide glutarique ou un analogue fonctionnel en dérivant, fournissant un composé alcool tertiaire de formule (15) et/ou l'une des formes tautomères en dérivant de formule (14A) ou (14B) ; 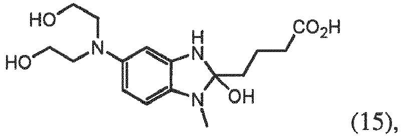 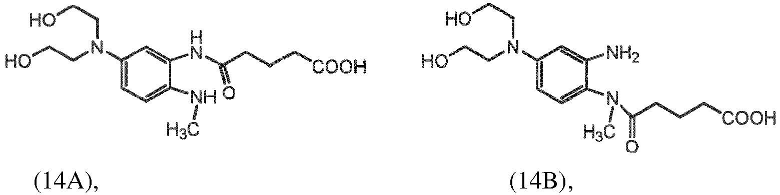 et
d] déshydratation et, éventuellement, estérification du produit obtenu à l'étape c), pour obtenir le composé de formule (11).

2. Le procédé selon la revendication 1, comprenant en outre la conversion du composé de formule (11) en bendamustine de formule (1) 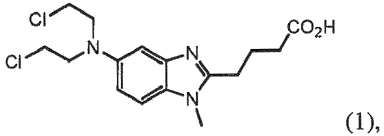 ou en un sel pharmaceutiquement acceptable de celle-ci.

3. Le procédé selon la revendication 1 ou 2, dans lequel la séquence de procédé des étapes a) à c) précitées ou, éventuellement, des étapes a) à d) précitées, est mise en oeuvre sans isolation ou purification des intermédiaires.

4. Le procédé selon l'une quelconque des revendications 1-3, dans lequel la réaction de l'étape a) est réalisée en présence d'acide borique.

5. Le procédé selon l'une quelconque des revendications 1-4, dans lequel l'une quelconque des étapes b), c) ou d) est réalisée dans un milieu réactionnel consistant en un alcool en C₁-C₄.

6. Le composé de formule (15), (14A), (14B) 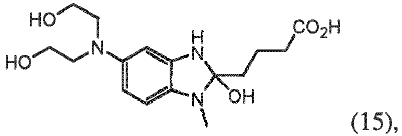 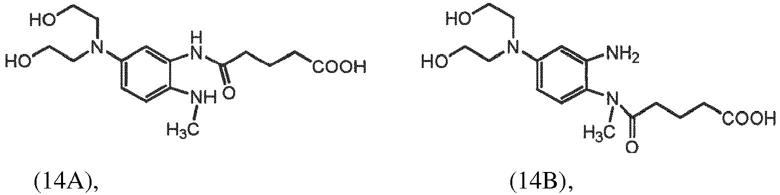 ou l'un de ses esters avec un alcool aliphatique en C₁-C₄ et/ou avec un acide carboxylique en C₁-C₁₀, et/ou n'importe lequel de ses sels d'addition acide.

7. Utilisation de l'un quelconque des composés de formule (15), (14A) et (14B) pour la préparation de bendamustine de formule (1), ou un sel pharmaceutiquement acceptable de celle-ci.

8. Le composé de formule (14) 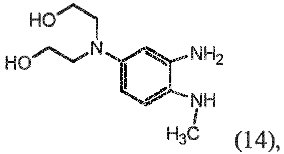 ou un sel d'addition acide de celui-ci.

9. Un procédé de préparation d'un composé de formule (14) comprenant les étapes suivantes :
a] obtention du composé de formule (13) par réaction du composé de formule (12), et
b] réduction du composé de formule (13).

10. Utilisation du composé de formule (14) pour la préparation de bendamustine de formule (1) ou d'un sel pharmaceutiquement acceptable de celle-ci.

11. Utilisation du composé de formule (12) ou (13) pour la préparation de bendamustine de formule (1), ou d'un sel pharmaceutiquement acceptable de celle-ci.

12. Le procédé selon l'une quelconque des revendications 1-5, dans lequel les groupes OH des chaines latérales bis-hydroxyéthylamino des composés (12), (13), (14), (15), et (11) sont protégés par un groupe OH-protecteur (X) adéquat, et dans lequel le groupe OH-protecteur est éliminé à un moment convenable lors de la conversion du composé de formule (11) en bendamustine de formule (1), ou en un sel pharmaceutiquement acceptable de celle-ci.

13. L'utilisation selon la revendication 10 ou 11, dans laquelle les groupes OH des chaines latérales bis-hydroxyéthylamino des composés (12), (13) ou (14) sont protégés par un groupe OH-protecteur (X) convenable et dans laquelle le groupe OH-protecteur est éliminé à un moment approprié.
